# EUROPEAN PATENT APPLICATION

(11) **EP 1 059 090 A1**
(43) Date of publication of application: **13.12.2000**
(21) Application number: 99906548.5
(22) Date of filing: 02.03.1999
(51) Int. Cl.: A61K 45/00, A61K 31/425

(54) **REMEDIES FOR BRAIN INFARCTION**

(30) Priority: 03.03.1998 JP 5024198
(71) Applicant: YAMANOUCHI PHARMACEUTICAL CO. LTD., Tokyo 103-8411 (JP)
(72) Inventor: OKADA, M., Yamanouchi Pharmaceutical Co., Ltd., Ibaraki 305-8585 (JP); TAKAHASHI, M., Yamanouchi Pharmaceutical Co.,Ltd, Ibaraki 305-8585 (JP); HAYASHIBE, S., Yamanouchi Pharmaceutical Co.,Ltd., Ibaraki 305-8585 (JP)
(74) Representative: Geering, Keith Edwin
(86) International application number: JP9900995
(87) International publication number: WO9944639

(57) **Abstract**

This invention is to provide remedies for cerebral infarction which contain as an active ingredient a compound having mGluR1 antagonism, pharmaceutical compositions which are remedies for cerebral infarction at the acute stage, pharmaceutical compositions which contain as an active ingredient a compound having selective mGluR1 antagonism, and pharmaceutical compositions wherein the compound having selective mGluR1 antagonism is 6-amino-N-cyclohexyl-N,3-dimethylthiazolo[3,2-a]benzoimidazole-2-carboxamide dihydrochloride.

Means for resolution Since a compound having mGluR1 antagonism showed an effect of reducing infarction volume in a rat cerebral infarction model, it was found out that mGluR1 antagonists are useful in preventing and treating cerebral infarction.

## Description

### Technical Field

This invention relates to novel medicaments as treatments for cerebral infarction, which contain a compound having mGluR1 antagonism.

### Background Art

Cerebral infarction is a condition in which neuronal cells fall into irreversible cell death caused by the formation of focal ischemic parts in the brain due to considerable reduction of focal cerebral blood flow affected by partial occlusion in brain blood vessels or reduction of perfusion pressure. Since infarction parts expand with the lapse of time and cause serious higher functional disorders, it is necessary to conduct treatment as soon as possible after the onset.

It has been revealed that not only exhaustion of energy due to reduced blood flow but also various processes such as neuronal cell toxicity of glutamate and free radicals are concerned in the neuronal cell death by ischemia.

The glutamate hypothesis is a dominant hypothesis regarding the neuronal cell death at the time of ischemia. Extracellular glutamate concentration increases at the time of ischemia (*Stroke*, 21, 1727 - 1733, 1990), and the glutamate excessively stimulates neuronal cells. It is considered that this phenomenon causes excess and continuous increase in the intracellular calcium concentration and thereby results in the death of neuronal cells.

Glutamate receptors have various subtypes such as NMDA receptors, AMPA receptors and kainate receptors.

Besides these subtypes, metabotropic glutamate receptors exist as kinds of glutamate receptors' subtypes (Neuropharmacology 34, 1-26 (1995)).

The metabotropic glutamate receptors consist of eight different subtypes as mGluR1-8. These are classified into three groups, Group I (mGluR1 and mGluR5), Group II (mGluR2 and mGluR3) and Group III (mGluR4, mGluR6, mGluR7 and GluR8), according to their sequence similarities, and pharmacological profiles.

Among the aforementioned glutamate receptors, antagonists of NMDA receptor and AMPA receptor can suppress neuronal cell death (*Ann. Neurol.,* 24, 543 - 551, 1988; *J*. *Pharmacol. Exp. Thr*., 276, 84 - 92, 1996), so that they are under clinical examinations regarding neuronal cell protection at the acute stage of cerebro-vascular accident.

Though metabotropic glutamate receptors are appeared to be a significant component in the cascades following excessive glutamate release during and after cerebral ischemia, their roles are not yet clear. Particularly, as the activation of mGluR1 and mGluR5 which is coupled to intracellular IP3 system increases intracellular calcium (Nature, 383, 89-92, 1996), continuous excess stimulation of these receptors may cause neuronal cell death.

Regarding the neuroprotective effect of compounds having mGluR1 antagonism in cerebral ischemia, Cozz et al. reported that intraventicular administration of AIDA ((RS-1-aminoindan-1,5-dicarboxylic acid) reduced the loss of the neuronal cells found in CA1 area in gerbils which exposed to 5 min of cerebral ischemia (Society for Neuroscience Abstracts, vol. 23, 788.2, 1997). However, Henrich-Noack et al. reported that 4C3HPG ((S)-4-carboxy-3-hydroxyphenylglycine), which is an antagonist at the Group I mGluRs and an agonist at Group II mGluRs, is effective, but 4CPG ((S)-4-carboxyphenylglycine), which is a selective Group I mGluRs antagonist is not effective in the same model (Society for Neuroscience Abstracts, vol. 23, 756.8, 1997).

One of the reasons for these discrepancy is considered to be due to the insufficient efficacy and selectively of mGluR1 antagonists used in these experiments. Therefore, it is considered that the neuroprotective effect of compounds having mGluR1 antagonism in cerebral ischemia is not clearly confirmed.

On the other hand, it is known that the neuronal cell death at the time of ischemia have a diversity such as acute neuronal cell death, delayed neuronal cell death or slowly progressive neuronal death (*Brain Hypoxia*, 10, p. 109 (1996)).

The aforementioned five minute-bilateral common carotid artery ligation model of Mongolian gerbil is a special model which reflects the morbid state of global cerebral ischemia caused by cardiopulmonary arrest in human. The global cerebral ischemia model is a model which can observe influences upon a specific ischemia-fragile neuronal cell by carrying out reperfusion after a short period (from 5 to 10 minutes) of global cerebral ischemic condition. In this model, cell death does not occur morphologically for several days after reperfusion, but a delayed neuronal cell death is observed in which a specific neuronal cell of hippocampus dies on the 4th day after ischemia (*Brain Res*., 239, 57 - 69 (1982)).

On the other hand, a cerebral infarction model induces acute neuronal cell death in which neuronal cells of striatum and cerebral cortex die several hours after occlusion (*J. Cereb. Blood Flow Metab.*, 1, 53 - 60 (1981) and thereby forms infarction foci.

Thus, the global cerebral ischemia model is a model for judging the effect to suppress delayed neuronal cell death which occurs following reperfusion after a short period of transient global ischemic invasion. In this model, infarction foci are not formed.

On the other hand, the cerebral infarction which is found clinically at the most high frequency is a focal cerebral ischemia caused by the occlusion of cerebral artery, in which cell death occurs immediately after onset of the disease and infarction foci are formed.

Accordingly, it is considered that the global cerebral ischemia model and cerebral infarction model are different from each other in terms of the regions and neuronal cells where disorders are induced, and their neuronal cell death mechanisms are also different.

Thus, it is considered that drug evaluation by the cerebral infarction model is the most proper method for confirming effects of drugs in human, because it can judge their effect to reduce infarction parts after focal ischemia attack without reopening blood flow.

As described above, the neuroprotective effects of mGluR1 antagonist in brain ischemia have not been fully confirmed to date. Moreover, previous reports on the neuroprotective actions of Group I antagonists are restricted to only their effects on delayed neuronal death according to their experimental methods.

Therefore, it is difficult to predict from previous information that mGluR1 antagonists will be able to reduce the infarct volume and be therapeutic agents for cerebral infarction. Thus, in order to evaluate the usefulness as therapeutic drugs, it is necessary to validate their efficacy in an appropriate animal model which closely resemble human ischemic stroke patients, and great concern has been directed toward such confirmation.

### Disclosure of the Invention

An object of the invention is to provide excellent remedies for cerebral infarction.

With the aim of achieving the above object, the present inventors have conducted intensive studies and found that a substance having selective mGluR1 antagonism is effective for a permanent middle cerebral artery (MCA) occlusion model which is most close to the morbid state of human cerebral infarction, thus accomplishing the invention.

Accordingly, the invention relates to remedies for cerebral infarction, which contain a substance having mGluR1 antagonism.

The following further describes the invention.

It relates to a pharmaceutical composition for treating cerebral infarction, which comprises a compound having mGluR1 antagonism, preferably to a pharmaceutical composition for treating cerebral infarction at the acute stage. More preferably, it relates to a pharmaceutical composition for treating cerebral infarction, which contains as the active ingredient a compound having selective mGluR1 antagonism, most preferably to a pharmaceutical composition for treating cerebral infarction, wherein the compound having selective mGluR1 antagonism is 6-amino-N-cyclohexyl-N,3-dimethylthiazolo[3,2-a]benzoimidazole-2-carboxamide dihydrochloride.

It also relates to a method for treating cerebral infarction, which comprises administering a therapeutically effective amount of a compound having mGluR1 antagonism, more preferably to a method for treating cerebral infarction at the acute stage. More preferably, it relates to a method for treating cerebral infarction, which comprises administering a therapeutically effective amount of a compound having selective mGluR1 antagonism, most preferably to a method for treating cerebral infarction, wherein the compound having selective mGluR1 antagonism is 6-amino-N-cyclohexyl-N,3-dimethylthiazolo[3,2-a]benzoimidazole-2-carboxamide dihydrochloride.

It also relates to the use of a compound having mGluR1 antagonism for the manufacture of a remedy for cerebral infarction, preferably to the use of a compound having mGluR1 antagonism for the manufacture of a remedy for cerebral infarction at the acute stage, more preferably to the use of a compound having selective mGluR1 antagonism for the manufacture of a remedy for cerebral infarction, most preferably to the use of 6-amino-N-cyclohexyl-N,3-dimethylthiazolo[3,2-a]benzoimidazole-2-carboxamide dihydrochloride for the manufacture of a remedy for cerebral infarction.

As defined in the foregoing, cerebral infarction is a condition in which neuronal cells fall into irreversible cell death caused by the formation of focal ischemic regions in the brain due to occlusion in brain blood vessels or reduction of perfusion pressure.

The acute stage of cerebral infarction means within 48 hours after onset of the disease.

Regarding the substance having mGluR1 antagonism to be used in the invention as the active ingredient of the remedies for cerebral infarction, its structure is not limited and may be either a peptide compound or a non-peptide compound, with the proviso that it is a compound having strong antagonism against the mGluR1 receptor.

Compounds described in the following documents or patents can be cited as examples of such mGluR1 antagonist.
Unexamined published Japanese patent application No. 8-169884
WO 95/25110
WO 96/15099
WO 96/15100
WO 97/05109
WO 97/05137
WO 98/06724
Japanese Patent Application No. 9-357552

Particularly preferred is a compound represented by the following general formula or a pharmaceutically acceptable salt thereof. 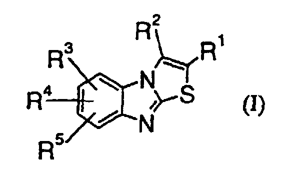 (In this formula, each symbol has the following meaning;
R¹:
   (1) -A¹-CO-N(R⁶)-R⁷,
   (2) -A¹-CO-A²-R⁸,
   (3) -A¹-CO-A³-N(R⁶)-R⁷,
   (4) -A²-O-A²-R⁹,
   (5) -A¹-R⁹,
   (6) -A¹-N(R⁶)-R⁷,
   (7) -A¹-N(R⁶)-CO-R⁷, or
   (8) -N(R¹⁰)-CO-O-R¹¹,
A¹ and A²: the same or different from each other and each represents a bond or a lower alkylene group which may be substituted by hydroxyl group,
R⁶ and R⁷: the same or different from each other and each represents hydrogen, a hydrocarbon group which may be substituted or heterocyclic group which may be substituted, wherein R⁶ and R⁷ together with the adjacent nitrogen atom may form a hetero ring which may have a substituent and have other hetero atom,
R⁸: hydrogen, a hydrocarbon group which may have a substituent or a hetero ring which may have a substituent, or a hydroxyl or lower alkyl-O- group,
A³: a lower alkylene group which may be substituted by hydroxyl group,
R⁹: hydrogen, a hydrocarbon group which may be substituted or heterocyclic group which may be substituted,
R¹⁰: hydrogen or a lower alkyl group,
R¹¹: a hydrocarbon group which may have a substituent or a heterocyclic group which may have a substituent,
R²: hydrogen, or a lower alkyl, halo-lower alkyl, hydroxy-lower alkyl, lower alkyl-O-lower alkyl, amino-lower alkyl or (mono- or di-lower alkyl-amino)-lower alkyl group,
R³, R⁴ and R⁵: the same or different from one another and each represents hydrogen, or a halo, lower alkyl, halo-lower alkyl, hydroxy-lower alkyl, hydroxy, lower alkyl-O-, cyano, -COOR¹⁴, acyl, acyl-O-, nitro or -A⁴-N(R¹²)-(R¹³) group,
R¹² and R¹³: the same or different from each other and each represents hydrogen or a lower alkyl group which may be substituted by acyl, aryl, hydroxyl, -COOR¹⁴ or heterocyclic group,
R¹⁴: hydrogen or lower alkyl group, and
A⁴: a bond or a lower alkylene group.)

In this connection, the aforementioned compounds preferable as the active ingredient of the medicaments of the invention include all of the compounds included in general formulae described in the aforementioned known documents and unpublished applications, and definitions of the broader terms or choices shown in these known documents can be employed directly as the definitions of the broader terms or choices of the invention. That is, in briefly describing, the term "lower" means a straight or branched carbon chain having from 1 to 6 carbon atoms, the lower alkyl means methyl, ethyl or the like C₁₋₆ alkyl, and the lower alkylene means methylene, ethylene or the like C₁₋₆ alkylene.

The "hydrocarbon group" which may have a substituent means (1)a lower alkyl (as defined above), (2) a lower alkenyl: vinyl, propenyl or the like C₂₋₆ alkenyl, (3) an aryl: phenyl, naphthyl, biphenyl or the like aromatic hydrocarbon ring group of from 6 to 14 members as a whole or (4) a saturated or unsaturated C₃₋₁₀ alicyclic group which may be condensed with benzene ring or crosslinked. More illustratively, 1) a C₃₋₈ cycloalkyl: preferably cyclopentyl, cyclohexyl or the like, 2) a C₃₋₈ cycloalkenyl: preferably cyclohexenyl group or the like, 3) a C₃₋₈ cycloalkyl condensed with benzene ring: preferably tetrahydronaphthyl, hexahydrobenzazepinyl or the like, 4) a C₃₋₈ cycloalkenyl condensed with benzene ring: preferably dihydronaphthyl, tetrahydrobenzazepinyl or the like or 5) a crosslinked saturated or unsaturated C₅₋₁₀ alicyclic group: preferably bicyclo[2.2.1]heptyl, bicyclo[3.2.1]octyl, bicyclo[3.3.1]nonyl, bicyclo[3.2.1]octenyl, adamantyl or the like.

These groups may have one or more substituents.

The "heterocyclic group" which may have a substituent means a monocyclic or bicyclic saturated or unsaturated hetero ring of from 4 to 14 members as a whole, that contains from 1 to 4 hetero atoms consisting of oxygen, sulfur and nitrogen atoms, wherein bicyclo forms and spiro forms are included.
(1) A 4- to 7-membered saturated or unsaturated monocyclic hetero ring group containing from 1 to 4 hetero atoms consisting of oxygen, sulfur and nitrogen atoms. Preferably pyrrolidinyl, piperidyl, piperazinyl, homopiperazinyl, hexahydroazepinyl, morpholinyl, pyridyl or the like group.
(2) A 4- to 7-membered saturated or unsaturated monocyclic hetero ring group containing from 1 to 4 hetero atoms consisting of oxygen, sulfur and nitrogen atoms, which is condensed with benzene ring. Preferably benzofuranyl, tetrahydrobenzofuranyl, indolyl, isoindolyl, benzazepinyl, tetrahydroquinolyl, tetrahydroisoquinolyl or the like.
(3) A saturated or unsaturated bicyclic hetero ring group of from 6 to 14 members as a whole, which contains from 1 to 4 hetero atoms consisting of oxygen, sulfur and nitrogen atoms. Preferably, decahydroquinolyl, 2-azabicyclo[2.2.2]heptyl, 1-azaspiro[4.5]decyl, 1-oxaspiro[4.5]decyl, 1,4-dioxaspiro[4.5]decyl, pyridoxazinyl, octahydrobenzoxazinyl or the like.

These may have one or more substituents.

The halo means fluorine, chlorine or the like halogen atom, and the mono- or di-lower alkyl-amino means an amino group substituted by 1 or 2 of the aforementioned lower alkyl.

The aryl is as defined in the aforementioned hydrocarbon group, and the acyl means a lower alkyl-CO- or an aryl-CO- and is preferably a lower alkyl-CO-.

The substituent of a group which may be substituted or have a substituent is as follows.

The substituent means a usual substituent for a group to be substituted, commonly used in this field, but is preferably selected from the class consisting of halo, lower alkyl, halo-lower alkyl, hydroxy, hydroxy-lower alkyl, lower alkyl-O-, oxo, acyl, acyl-O-, COOH, lower alkyl-O-CO-, lower alkyl-O-lower alkyl, NO₂, cyano, NH₂, mono- or di-lower alkyl-amino, phthalimido and the like groups. The number of substituents is not particularly limited so far as it is substitutable number, but is preferably from 1 to 4.

In addition, there is a case in which the active ingredient of the medicaments of the invention forms a salt with inorganic acids such as hydrochloride acid, organic acids such as fumaric acid, inorganic bases such as sodium or organic bases such as diethanolamine, or forms a quaternary ammonium salt, and pharmaceutically acceptable salts of the aforementioned compounds are included in the active ingredient of the invention. Also included in the active ingredient of the invention are all of various isomers in isolated or mixed forms, hydrates, solvates and various crystal forms.

### Production Methods

The substance having mGluR1 antagonism to be used in the invention, which is disclosed in the aforementioned patent application, can be produced by the method described in the specification of the patent application, and other substances having mGluR1 antagonism can be produced in the usual way.

Typical production methods of particularly preferable compounds are shown below.

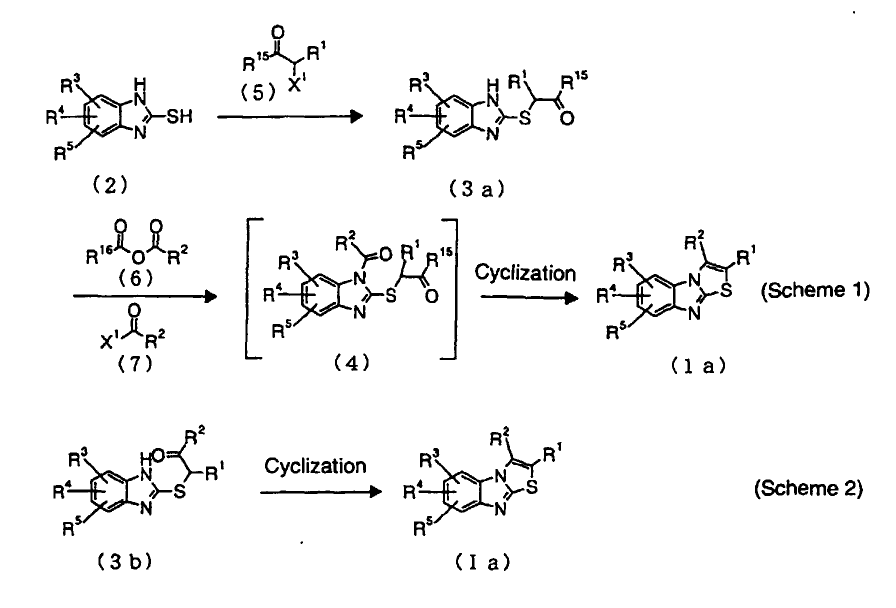

(In the above formulae, R¹ to R⁵ are as defined in the foregoing, R¹⁵ and R¹⁶ are lower alkyl groups, and X¹ is a halo.)

Thiazolo[3,2-a]benzoimidazole (1a) as the basic nucleus can be produced by the conventional method shown in the scheme 1 or 2. That is, it can be produced by allowing a 2-mercaptobenzoimidazole (2) to react with an α-haloketone (5) at room temperature or under a heating condition in an alcohol solvent such as ethanol or methanol or in an inert solvent such as tetrahydrofuran, dimethylformamide, acetone or acetonitrile, in the presence of a base such as sodium hydroxide, potassium hydroxide, sodium hydride or potassium carbonate or under a neutral condition, thereby obtaining a compound (3a) which is subsequently heated in the presence of an acid halide, acid anhydride or mixed acid anhydride (6) and a base such as pyridine or sodium acetate. Also, it can be produced by subjecting the compound (3a) to N-acylation using reaction-corresponding amounts of an acid halide (7) and the acid anhydride or mixed acid anhydride (6) in the presence of pyridine, triethylamine or the like base, thereby obtaining a compound (4) which is subsequently isolated and then heated in acetonitrile or the like inert solvent in the presence of pyridine or the like base. Alternatively, it can be produced by carrying out dehydration cyclization of a compound (3b) shown in the formula 2 in concentrated sulfuric acid or the like strong acid, or in acetic acid solvent using sulfuric acid or the like catalyst, at room temperature or under heating.

In addition, an amino group can be added to the 6-position of thiazolo[3,2-a]benzoimidazole by nitrating the 6-position under usual nitration conditions and then reducing it by the method shown in the production example which will be described later. The pharmaceutical preparation which contains one or two or more of the invention compounds or pharmaceutically acceptable salts thereof as the active ingredient to be used in the invention is prepared using generally used pharmaceutical carriers, fillers and other additives.

The carriers and fillers for use in the pharmaceutical preparation may be either in the solid or liquid form, and their examples include lactose, magnesium stearate, starch, talc, gelatin, agar, pectin, gum arabic, olive oil, sesame oil, cacao butter, ethylene glycol and other generally used materials.

In general, its administration is carried out preferably using injections by intravenous injection, intramuscular injection or the like administration, but it may also be parenteral administration in the form of suppositories, percutaneous preparations and the like, or oral administration in the form of tablets, pills, capsules, granules, powders, solutions and the like. The dose is optionally decided by taking into consideration symptoms, age, sex and the like of each patient to be treated, but the preparation is usually administered by intravenous injection within the range of from 0.05 to 50 g, preferably from 1 to 20 g, per day per adult by dividing the daily dose into 1 to several doses per day, or by continuous intravenous injection within the range of from 1 to 24 hours per day. Alternatively, it may be orally administered within the range of from 1 to 50 g per day per adult by dividing the daily dose into 1 to several doses per day. Since the dose varies under various conditions as a matter of course as described in the foregoing, a smaller dose than the above range may be sufficient enough in some cases.

The injections for parenteral administration includes aseptic aqueous or non-aqueous solutions, suspensions and emulsions. Examples of the aqueous solutions and suspensions include distilled water for injection use and physiological saline. Examples of the non-aqueous solutions and suspensions include propylene glycol, polyethylene glycol, olive oil or the like plant oil, ethanol and the like alcohols, polysorbate 80 and the like. Such a composition may further contain auxiliary agents such as a tonicity agent, an antiseptic, a moistening agent, an emulsifying agent, a dispersing agent, a stabilizing agent (e.g., lactose) and a solubilization assisting agent (e.g., glutamic acid or aspartic acid. They are sterilized for example by filtration through a bacteria retaining filter, blending of a germicide or irradiation. Alternatively, they may be used by firstly making into sterile solid compositions and dissolving them in sterile water or a sterile solvent for injection use prior to their use.

The solid composition for use in the oral administration according to the invention is used in the tablets, powders, granules and the like forms. In such a solid composition, one or more active substances are mixed with at least one inert diluent such as lactose, mannitol, glucose, hydroxypropylcellulose, microcrystalline cellulose, starch, polyvinyl pyrrolidone or aluminum magnesium silicate. In the usual way, the composition may contain other additives than the inert diluent, such as magnesium stearate or the like lubricant, calcium cellulose glycolate or the like disintegrating agent, lactose or the like stabilizing agent and glutamic acid, aspartic acid or the like solubilization assisting agent. If necessary, tablets or pills may be coated with a film of a gastric or enteric substance such as sucrose, gelatin, hydroxypropylcellulose, hydroxypropylmethylcellulose phthalate or the like.

The liquid composition for oral administration includes pharmaceutically acceptable emulsions, solutions, suspensions, syrups, elixirs and the like and contains a generally used inert diluent such as purified water or ethyl alcohol. In addition to the inert diluent, this composition may also contain a moistening agent, a suspending agent and the like auxiliary agents, as well as sweeteners, flavors, aromatics and antiseptics.

### Examples

Next, the invention is described further in detail based on examples, though the invention is not limited these examples.

### Example (Production of pharmaceutical preparation for intravenous use)

A 6 g portion of the compound A and 27 g of sodium chloride were dissolved in about 1,800 ml of water for injection use, and the solution was adjusted to a total volume of 3,000 ml by adding water for injection use. This solution was filtered through a membrane filter of 0.22 µm in pore size to prepare injections.

The following describes pharmacological test methods and pharmacological effects related to the invention.

### Cerebral infarction suppression effect of 6-amino-N-cyclohexyl-N,3-dimethylthiazolo[3,2-a]benzoimidazole-2-carboxamide dihydrochloride (compound A)

### 1. Selectivity

### (Cell culture)

NIH3T3 cells expressed mGluR1 α and mGluR5a individually were cultured in DMEM medium containing 10% dialyzed fetal bovine serum and 100 units/ml, 0.1 mg/ml streptomycin sulfate. CHO cells which expressed mGluR2, R3, R4, R6 and R7 individually were cultured in DMEM medium containing 10% dialyzed fetal bovine serum, 100 units/ml, 0.1 mg/ml streptomycin sulfate and 2 mM glutamine.

### (Measurement of intracellular calcium concentration)

Intracellular calcium concentration in the mGluR1 α-expressing cells was measured as previously described (*Nature*, 383, 89 - 92, 1996) using a fluorescence spectrophotometer.

### (Measurement of phosphatidylinositol (PI) hydrolysis)

Hydrolysis of phosphatidylinositol was measured as previously described (*Nature*, 383, 89 - 92, 1996) using the mGluR1 α-expressing cells incorporated ³H-inositol.

### (Measurement of intracellular cAMP)

Using the cells expressing mGluR2, R3, R4, R6 and R7 cAMP formation induced by forskolin in the presence of IBMX was measured using a cAMP measuring kit as previously described (*Neuron*, 8, 169 - 179, 1992).

### 2. Cerebral infarction suppression action

### (permanent MCA occlusion model)

Compound A was dissolved in physiological saline and adjusted to 6 mg/3 ml.

In accordance with the known method (*J. Pharmacol. Exp. Thr*., 276, 84 - 92, 1996), left MCA of a Fischer-344 rat was subjected to permanent occlusion and, 5 minutes thereafter, the compound A was intravenously administered continuously for 24 hours at a rate of 6 mg/a dose volume of 3 ml/kg/h under no anesthesia and no restriction. After completion of the administration, the animal was decapitated to excise the brain which was subsequently stained with 2,3,5-triphenyltetrazolium hydrochloride (TTC) to measure infarction volume.

### Results

### 1. Selectivity

Compound A up to 100 µM has no agonist and antagonist activities against mGlu R2, R3, R4, R6 and R7. And also compound A up to 10 µM has no agonist and antagonist activities against mGluR5.

Consequently, it was confirmed that the compound A has no activity against other metabotropic glutamate Groups (Group II and Group III).

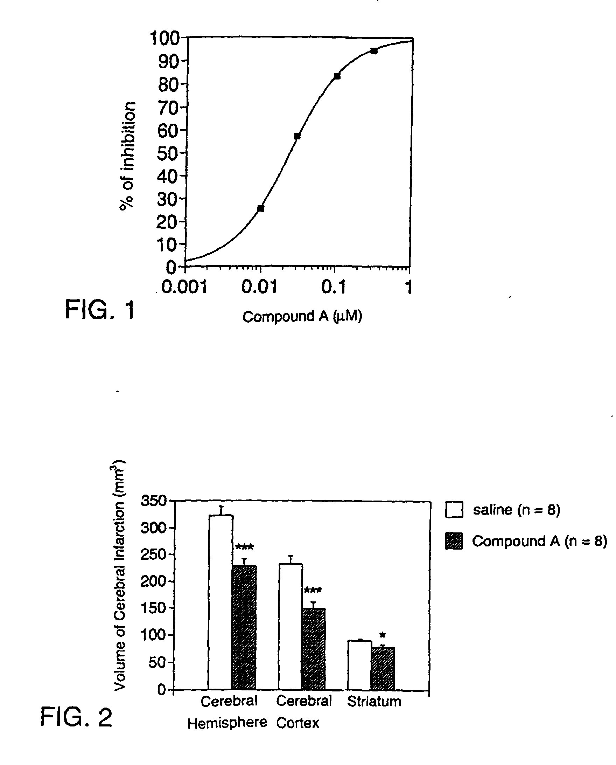

Fig. 1 shows dose-dependently inhibiting effect of compound A against mGluR1α. As shown in Fig. 1, compound A dose-dependently inhibited 100 µM glutamate-induced PI formation against mGluR1α. The IC₅₀ value of compound A was 24 nM.

### 2. Cerebral infarction volume reducing action

The results are shown in Fig. 2.

In Fig. 2, cerebral infarction volumes in cerebral hemisphere, cerebral cortex and striatum after administration of the compound A are compared with controls.

As shown in Fig. 2, the compound A reduced the cerebral infarction volume significantly, and its suppression ratios in cerebral hemisphere and cerebral cortex were 29% and 36%, respectively.

As the results of the above tests, a compound having selective and strong antagonism against mGluR1 showed the cerebral infarction volume-reducing effect in a cerebral infarction animal model showing infarction regions caused by focal ischemia. Based on these results, efficacy of a compound having mGluR1 antagonism in treating cerebral infarction was proved, and its usefulness in treating cerebral infarction at the acute stage was confirmed.

### Production Example 1

### 6-Amino-N-cyclohexyl-N,3-dimethylthiazolo[3,2-a]benzoimidazole-2-carboxamide dihydrochloride

At room temperature, an aqueous solution (50 ml) of sodium hydrosulfite (12.5 g) was added to a THF (80 ml)methanol (30 ml) solution of N-cyclohexyl-N,3-dimethyl-6-nitrothiazolo[3,2-a]benzoimidazole-2-carboxamide (5.35 g), and the mixture was stirred at the same temperature for 12 hours. Next, concentrated hydrochloric acid (10 ml) was added, followed by heating under reflux for 1 hour. Next, THF and methanol were evaporated under a reduced pressure, and the residue was diluted with water and then neutralized with 28% aqueous ammonia. This was extracted with ethyl acetate, washed with water and saturated brine and dried over anhydrous sodium sulfate, and then the solvent was evaporated under a reduced pressure. The resulting residue was purified by a column chromatography (eluent: chloroform:methanol = 20:1), made into hydrochloride and then recrystallized from methanol-ethyl acetate to obtain the title compound (3.78 g) as light brown crystals.
NMR: 8.11 (d, 1H), 7.84 (d, 1H), 7.41 (dd, 1H), 4.20 - 5.75 (br), 3.80 - 4.20 (br, 1H), 2.94 (s, 3H), 2.71 (s, 3H), 1.50 - 1.85 (m, 7H), 1.22 - 1.40 (m, 2H), 1.02 - 1.18 (m, 1H)
MS (FAB): 343 (M + 1).

## Claims

1. A pharmaceutical composition for treating cerebral infarction, which comprises a compound having mGluR1 antagonism as an active ingredient.

2. The pharmaceutical composition according to claim 1, which is for treating cerebral infarction at the acute stage.

3. The pharmaceutical composition according to claim 1, which comprises a compound having selective mGluR1 antagonism as an active ingredient.

4. The pharmaceutical composition according to claim 3, wherein the compound having selective mGluR1 antagonism is 6-amino-N-cyclohexyl-N,3-dimethylthiazolo[3,2-a]benzoimidazole-2-carboxamide dihydrochloride.
